(19) Europäisches Patentamt
European Patent Office
Office européen des brevets

(11) EP 0 995 449 A1

(12) EUROPÄISCHE PATENTANMELDUNG

(43) Veröffentlichungstag:
**26.04.2000 Patentblatt 2000/17**

(51) Int. Cl.$^7$: **A61L 27/00**

(21) Anmeldenummer: **98811048.2**

(22) Anmeldetag: **21.10.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Sulzer Orthopädie AG**
**6340 Baar (CH)**

(72) Erfinder: **Schaffner, Silvio**
**8254 Basadingen (CH)**

(74) Vertreter: **Sulzer Management AG**
**KS/Patente/0007**
**Zürcherstrasse 14**
**8401 Winterthur (CH)**

(54) **UHMW-Polyethylen für Implantate**

(57) UHMW-Polyethylen für Implantate, die für eine Sterilisation mittels γ-Strahlen oder Elektronenstrahlen vorgesehen sind, werden bei der Herstellung des Rohmaterials mit Vitamin E dotiert, welches nach der Bestrahlung die freien Radikalen schneller als der Sauerstoff der Umgebung abbindet und so eine Oxidation und Alterung verhindert. Ein pulverförmiges Ausgangsmaterial wird auf seiner Oberfläche mit einer Flüssigkeit benetzt, die eine passende Menge an Vitamin E aufweist, um eine Konzentration K von Vitamin E 0,01 % < K < 1 % an den Polyethylenteilchen zu erreichen. Nach dem Verdampfen der Flüssigkeit wird das PE-Pulver bei Temperaturen um 180°C - 240°C und Drücken von 2 - 10 MPa zu Blöcken verpresst oder Stangen verarbeitet.

EP 0 995 449 A1

## Beschreibung

Die Erfindung handelt von UHMW-Polyethylen für Implantate, die für eine Sterilisation mittels γ-Strahlen oder Elektronenstrahlen vorgesehen sind.

Nur wenige Firmen haben sich auf die Herstellung von UHMW-Polyethylen für medizinische Zwecke spezialisiert. Eine übliche Herstellung benutzt Pulver oder Granulat, welches bei Temperaturen um 180°C - 240°C und Drükken um 2 - 10 MPa zu Fertigteilen oder zu Blöcken verpresst wird oder zu Stangen extrudiert wird, aus denen Implantatteile, beispielsweise die Lagerschalen von künstlichen Hüftgelenken oder künstlichen Kniegelenken gefertigt werden.

Eine übliche Weiterverarbeitung der Implantatteile besteht darin, die Teile in einer Schutzgasatmosphäre beispielsweise in Stickstoff in einem Beutel einzuschweissen und in diesem Beutel durch Bestrahlung mittels γ-Strahlen oder Elektronenstrahlen zu sterilisieren. In diesem Zustand sind die Implantatteile lagerfähig und stehen jederzeit für eine Implantation zu Verfügung.

Die Bestrahlung mittels γ-Strahlen oder Elektronenstrahlen bringt neben der Sterilisation den Effekt, dass im Polyethylen auch Kettenstrukturen mit freien Radikalen entstehen, welche zu einer Vernetzung und/oder zu einer Oxidation führen wenn Sauerstoffatome verfügbar sind.

Sauerstoff ist zum einen im Polyethylen vorhanden und kann zum anderen langsam in das Polyethylen hineindiffundieren, was dazu führt, dass nach einigen Jahren eine Versprödung des Materials einsetzt, welche die mechanischen Eigenschaften reduziert und zu einem erhöhten Verschleiss führt.

In der Schrift CS 221403 vom 15.09.1982 wird über eine nicht gesundheitsschädliche Stabilisierung von Polyolefinen gegen Thermooxidation und gegen Photooxidation berichtet. Verschiedene Polyolefine altern langsamer unter natürlichen Umweltbedingungen, wenn man ihnen während der Herstellung ein Tocopherol beimischt. Im Gegensatz zu anderen Antioxidanzien wie Derivaten von Phenolaminen und Phosphiden, die gesundheitlich bedenklich sein können, wird vorgeschlagen Polyolefinen, die in der Lebensmittelindustrie, im Gesundheitswesen oder als Implantate verwendet werden, Tocopherol zuzumischen, um eine langsamere Alterung zu erreichen. Dabei sollen Gewichtsanteile von 0,01 % bis 5 % an Tocopherol bei den Polyolefinen die Thermooxidation bei der Herstellung des Materials und die Photooxidation des Materials unter natürlichen Umweltbedingungen verlangsamen.

Hier setzt die Erfindung ein. Sie hat die Aufgabe zu verhindern, dass an mit γ- oder Elektronenstrahlen bestrahlten Implantatteilen eine Oxidation in grösserem Ausmass stattfinden kann.

Diese Aufgabe wird mit den Kennzeichen vom unabhängigen Anspruch 1 dadurch gelöst, dass im Polyethylen Vitamin E dispers eingelagert ist mit einer Konzentration K von $0{,}01\,\% < K < 1\,\%$, um eine langfristige Versprödung und einen damit einsetzenden Verschleiss an Kontaktstellen zu verhindern.

Es hat sich nämlich gezeigt, dass dispers verteiltes Vitamin E die freien Radikalen schneller als eindiffundierender Sauerstoff absättigen kann und somit ein Fortschreiten der Oxidation verhindert. Es hat sich ebenfalls gezeigt, dass ein zu grosser Prozentsatz an eingelagerten Vitamin E beispielsweise über 1 % ebenfalls zu einer Verschlechterung der mechanischen Eigenschaften führt, indem sich der E-Modul, die Zugfestigkeit und die Kerbschlagzähigkeit des Materials verschlechtern. Andererseits kann die Oxidation bereits bei einem Anteil an Vitamin E der grösser als 0,01 % ist, spürbar beeinträchtigt werden. Es kommt eben darauf an, die disperse Verteilung so gleichmässig wie möglich und mit einem Aufwand, der sich wirtschaftlich noch rechtfertigen lässt, vorzunehmen.

Die abhängigen Patentansprüche 2 bis 6 und 8 bis 12 entsprechen Weiterbildungen der Erfindung. Je besser es gelingt gleichmässig kleine Teilchen zu erzeugen und gleichmässig an ihrer Oberfläche mit Vitamin E zu dotieren, desto enger und wirkungsvoller sind die erreichbaren Grenzen für eine mittlere Konzentration von Vitamin E, die in einer verbesserten Näherung $0{,}1\,\% < \overline{K} < 0{,}4\,\%$ und bei verbesserter Auslese und Benetzung der PE-Teilchen $0{,}1\,\% < \overline{K} < 0{,}2\,\%$ betragen kann.

Sehr kleine Teilchen aus UHMW-Polyethylen haben eine relativ grosse Oberfläche im Verhältnis zu ihrem Volumen. Wenn es gelingt diese Teilchen sehr fein zu mahlen, was mit Ultraschallmühlen, die mit sich kreuzenden Schallfronten kleinste Teilchengrössen erzeugen können, möglich ist, dann kann Vitamin E, welches in einer Flüssigkeit als Suspension gelagert ist oder welches in einem Lösungsmittel enthalten ist, in einer kleinen und beherrschbaren Konzentration mit der Flüssigkeit an den Polyethylenteilchen angelagert werden. Je ähnlicher die Polyethylenteilchen im Durchmesser sind desto ähnlicher ist auch ihr Faktor für das Verhältnis von Oberfläche zu Volumen und die Gewissheit bei einer bestimmten Oberflächenspannung der Flüssigkeit gleiche Mengen von Vitamin E an den Teilchen anzulagern, wenn die Flüssigkeit verdampft wird, was auch bei niederen Temperaturen im Vakuum möglich ist. Als Lösungsmittel für Vitamin E sind zum Beispiel Alkohole geeignet. Ein so entstandenes Feinstpulver welches gleichmässig mit einer niedrigen Konzentration von Vitamin E dotiert ist, lässt sich sofort oder nach Zwischenlagerung in einer neutralen Atmosphäre zu den erwähnten Bedingungen in kompakte Blöcke und Tafeln verpressen. Das Vitamin E ist dispers eingelagert und erfüllt seine Funktion erst, wenn fertig bearbeitete Implantatteile zur Sterilisation bestrahlt worden sind. Die nach der Bestrahlung noch vorhandenen freien Radikale werden schneller vom Vitamin E als vom Sauerstoff abgesättigt und verhindern so eine frühzeitige Oxidation und Alterung. Natürliches Vitamin E enthält neben dem

Hauptbestandteil α-Tocopherol noch β-Tocopherol und weitere Isomere, die eine etwas geringere Wirkung als Antioxidans haben.

α-Tocopherol mit der Strukturformel

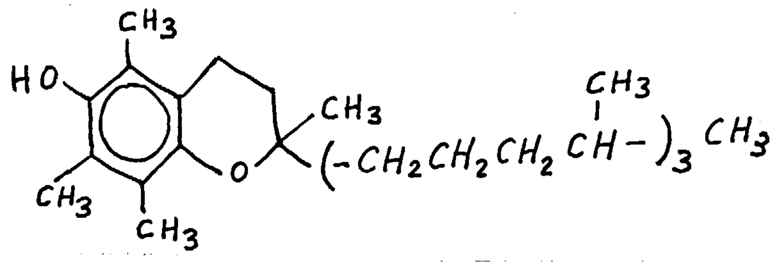

entspricht einem handelsüblichen Vitamin E in Form einer pasteusen Masse, die lösbar ist in organischen Lösemitteln wie Alkohole, Ketone und flüssigen Alkanen z.B. Ethanol, Aceton oder n-Heptan. Bei der Festlegung der Lösungskonzentration und -menge muss die Oberfläche der Polyethylenteilchen und die des Mischgefässes berücksichtigt werden, um die PE-Teilchen gleichmässig und in einer ihrem Volumen entsprechenden Konzentration $\overline{K}$ von Vitamin E mit Lösungsmittel und Vitamin E zu benetzen. Das Verdampfen vom Lösungsmittel kann in einem Autoklav durch Aufrechterhalten eines Vakuums vorgenommen werden.

Um nicht gewollten Reaktionen des Vitamins E vorzubeugen, welches auf eine grosse Oberfläche verteilt ist, kann man die mit Vitamin E belegten Polyethylenteilchen im Vakuum oder in einer Schutzgasatmosphäre lagern.

**Patentansprüche**

1. UHMW-Polyethylen für Implantate, die für eine Sterilisation mittels γ-Strahlen oder Elektronenstrahlen vorgesehen sind, dadurch gekennzeichnet, dass im Polyethylen Vitamin E dispers eingelagert ist mit einer Konzentration K von 0,01 % < K < 1 %, um eine langfristige Versprödung und einen damit einsetzenden Verschleiss an Kontaktstellen zu verhindern.

2. UHMW-Polyethylen nach Anspruch 1, dadurch gekennzeichnet, dass die mittlere Konzentration von Vitamin E 0,1 % < $\overline{K}$ < 0,4 % beträgt.

3. UHMW-Polyethylen nach Anspruch 1, dadurch gekennzeichnet, dass die mittlere Konzentration von Vitamin E 0,1 % < $\overline{K}$ < 0,2 % beträgt.

4. UHMW-Polyethylen nach einem der Ansprüche 1 bis 3, dadurch gekennzeichnet, dass es sich bei dem Vitamin E um α-Tocopherol mit einer Strukturformel

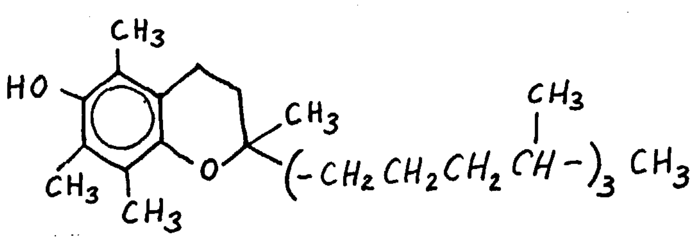

handelt.

5. UHMW-Polyethylen nach einem der Ansprüche 1 bis 4, dadurch gekennzeichnet, dass es durch spanende Formgebung zu einem Implantat weiterverarbeitet ist, welches innerhalb einer Umhüllung in einer Schutzgasatmosphäre eingeschlossen ist und zur Sterilisation einer γ-Strahlung ausgesetzt wurde.

**6.** UHMW-Polyethylen nach Anspruch 5, dadurch gekennzeichnet, dass die Strahlungsmenge mindestens 2,5 Mrad beträgt.

**7.** Verfahren zum Herstellen eines UHMW-Polyethylens nach einem der Ansprüche 1 bis 6, dadurch gekennzeichnet, dass

- in einem ersten Schritt Pulver oder Granulat aus UHMW-Polyethylen mit einer Flüssigkeit gemischt wird, die Vitamin E in einer vorgegebenen Menge enthält,
- in einem zweiten Schritt die Flüssigkeit verdampft wird, um das Vitamin E in einer vorgegebenen Konzentration auf den Polyethylenteilchen anzulagern,
- in einem dritten Schritt die Polyethylenteilchen bei Temperaturen um 180°C - 240°C und Drücken um 2 - 10 MPa zu Blöcken verpresst oder zu Stangen extrudiert werden.

**8.** Verfahren nach Anspruch 7, dadurch gekennzeichnet, dass die Teilchen aus UHMW-Polyethylen annähernd gleiche Grösse haben, um bei einem bestimmten Verhältnis von Oberfläche zu Volumen der Teilchen eine vorgegebene Konzentration von Vitamin E bei allen Teilchen anzulagern.

**9.** Verfahren nach einem der Ansprüche 7 oder 8, dadurch gekennzeichnet, dass die Flüssigkeit ein Lösungsmittel für Vitamin E ist, beispielsweise ein Alkohol.

**10.** Verfahren nach einem der Ansprüche 7 oder 8, dadurch gekennzeichnet, dass die Flüssigkeit eine Trägerflüssigkeit ist, in der das Vitamin E als Suspension eingelagert ist.

**11.** Verfahren nach einem der Ansprüche 7 bis 10, dadurch gekennzeichnet, dass einzelne Schritte in einer Schutzgasatmosphäre ohne Sauerstoff ausgeführt werden.

**12.** Verfahren nach einem der Ansprüche 7 bis 11, dadurch gekennzeichnet, dass zwischen dem zweiten und dritten Schritt, eine Lagerung in einer Schutzgasatmosphäre durchgeführt wird.

Europäisches Patentamt

# EUROPÄISCHER RECHERCHENBERICHT

Nummer der Anmeldung
EP 98 81 1048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br><br>Y | EP 0 613 923 A (HOECHST AG)<br>7. September 1994<br>* das ganze Dokument *<br>--- | 1-4<br><br>1-12 | A61L27/00 |
| Y | US 5 650 485 A (SUN DEH-CHUAN ET AL)<br>22. Juli 1997<br>* Spalte 5, Zeile 23 - Spalte 6, Zeile 34 *<br>* Spalte 7, Zeile 37 - Spalte 8, Zeile 14 *<br>--- | 1-12 | |
| D,X | CS 221 403 B (DOLEZEL BRETISLAV ET AL.)<br>29. April 1983<br>* das ganze Dokument *<br>--- | 1-4 | |
| X | EP 0 542 108 A (HOFFMANN LA ROCHE)<br>19. Mai 1993<br>* Seite 2, Zeile 43 - Zeile 49 *<br>* Ansprüche 1,2,6,8 *<br>----- | 1-4 | |

RECHERCHIERTE SACHGEBIETE (Int.Cl.6)
A61L

Der vorliegende Recherchenbericht wurde für alle Patentansprüche erstellt

| Recherchenort | Abschlußdatum der Recherche | Prüfer |
|---|---|---|
| DEN HAAG | 19. März 1999 | Heck, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 98 81 1048

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-03-1999

| Im Recherchenbericht angeführtes Patentdokument | Datum der Veröffentlichung | Mitglied(er) der Patentfamilie | Datum der Veröffentlichung |
|---|---|---|---|
| EP 0613923 A | 07-09-1994 | DE 4306593 A | 08-09-1994 |
| | | JP 2566112 B | 25-12-1996 |
| | | JP 6299011 A | 25-10-1994 |
| US 5650485 A | 22-07-1997 | US 5449745 A | 12-09-1995 |
| | | US 5414049 A | 09-05-1995 |
| | | US 5728748 A | 17-03-1998 |
| | | AT 171078 T | 15-10-1998 |
| | | AU 684500 B | 18-12-1997 |
| | | AU 6436494 A | 20-12-1994 |
| | | DE 9490446 U | 20-02-1997 |
| | | DE 69413395 D | 22-10-1998 |
| | | DE 69413395 T | 04-02-1999 |
| | | EP 0701453 A | 20-03-1996 |
| | | EP 0847765 A | 17-06-1998 |
| | | ES 2122260 T | 16-12-1998 |
| | | WO 9427651 A | 08-12-1994 |
| | | JP 2836965 B | 14-12-1998 |
| | | JP 8509148 T | 01-10-1995 |
| | | US 5543471 A | 06-08-1996 |
| CS 221403 B | 29-04-1983 | KEINE | |
| EP 0542108 A | 19-05-1993 | US 5308549 A | 03-05-1994 |
| | | AT 158603 T | 15-10-1997 |
| | | DE 69222394 D | 30-10-1997 |
| | | DE 69222394 T | 29-01-1998 |
| | | DK 542108 T | 04-05-1998 |
| | | ES 2108067 T | 16-12-1997 |
| | | JP 5320411 A | 03-12-1994 |

EPO FORM P0461

Für nähere Einzelheiten zu diesem Anhang : siehe Amtsblatt des Europäischen Patentamts, Nr.12/82